(19) Europäisches Patentamt
European Patent Office
Office européen des brevets

(11) Veröffentlichungsnummer: **0 073 326**
**B1**

# (12) EUROPÄISCHE PATENTSCHRIFT

(45) Veröffentlichungstag der Patentschrift: **23.10.85**

(51) Int. Cl.⁴: **C 07 C 120/10**, C 07 C 121/20, C 07 C 121/52, B 01 J 21/02

(21) Anmeldenummer: **82106327.8**

(22) Anmeldetag: **15.07.82**

## (54) Verfahren zur Herstellung von Nitrilen aus Formamiden.

(30) Priorität: **05.08.81 DE 3130979**

(43) Veröffentlichungstag der Anmeldung: **09.03.83 Patentblatt 83/10**

(45) Bekanntmachung des Hinweises auf die Patenterteilung: **23.10.85 Patentblatt 85/43**

(84) Benannte Vertragsstaaten: **BE CH DE FR GB IT LI**

(56) Entgegenhaltungen:
**DE - A - 1 954 827**
**DE - B - 1 286 001**
**DE - C - 1 117 121**
**US - A - 2 904 579**
**SOVIET INVENTIONS ILLUSTRATED Week D 43, 2 December 1981 Section E17**
**Patents Abstracts of Japan Band 5, Nr. 194, 10. Dezember 1981**

(73) Patentinhaber: **BASF Aktiengesellschaft, Carl-Bosch-Strasse 38, D-6700 Ludwigshafen (DE)**

(72) Erfinder: **Magnussen, Peter, Dr., Professor-Dillinger-Weg 25, D-6702 Bad Duerkheim (DE)**
Erfinder: **Trauzettel, Wulf, Dr., Osloer Weg 36, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 073 326 B1

BUNDESDRUCKEREI BERLIN

**Beschreibung**

Die Erfindung betrifft ein Verfahren zur Herstellung von Nitrilen durch Umsetzung von N-Formylaminen bei 400 bis 700°C in Gegenwart eines oberhalb 10 bis 50 Gewichtsprozent Boroxid enthaltenden Trägerkatalysators.

Es ist aus der deutschen Patentschrift 482 943 bekannt, daß sich beim Überleiten von Dämpfen ameisensaurer primärer Amine, ihrer Derivate oder Substitutionsprodukte bei erhöhten Temperaturen an hochporösen Katalysatoren (Silikagel, Aktivkohle) Nitrile darstellen lassen. Statt der Dämpfe des Salzes sind ebenso Gemische von Ameisensäureestern und den Aminen oder Formamide verwendbar.

Die deutsche Patentschrift 1 086 710 beansprucht ein Verfahren zur Herstellung von aliphatischen $\alpha,\omega$-Aminonitrilen ($H_2N-(CH_2)_n-CN$) und $\alpha,\omega$-Dinitrilen ($NC-(CH_2)_n-CN$) aus den entsprechenden Bis-formyl-amino-Verbindungen ($OHC-NH-(CH_2)_n-NH-CHO$) bei 400 bis 600°C mittels speziellen Kieselsäure- oder Silikat-Katalysatoren. $TiO_2$-Dotierung des Kieselgels begünstigt die Nitrilbildung ebenso wie die Gegenwart von Ammoniak im Reaktionsraum. Man erhält kein reines Dinitril, sondern ein Gemisch der 2 vorgenannten Komponenten. Es wird beschrieben, daß nur spezielle Porosität besitzende Katalysatoren von Bedeutung sind, da schon geringe Änderungen (Beispiel 1, Zeilen 54—61) die Ausbeute an Korksäurenitril von 27,1% auf 13,3% fällt. Ebenso wesentlich ist eine bestimmte Austauschacidität (≧ pH 3). Als Aktivatoren werden Metalloxide der III. bis VI. Gruppe des Periodischen Systems erwähnt, in einer Menge von 0,1 bis 10 Gewichtsprozent, doch keine Einzelmetalle außer Titan genannt.

Die deutsche Patentschrift 1 117 121 beschreibt einen speziellen Kieselgel- oder Silikat-Katalysator, den Temperaturbereich von 460—560°C, die Verwendung von Inertgasen, den Einsatz eines vorgeschalteten Formylierungsreaktors, die Anwendung eines Aminüberschusses und die Verwertung von durch Luftoxidation regenerierten Katalysatoren. Auch hier wird nachträglich die Bedeutung von speziellen Katalysatoren mit besonderen Werten von Porenradius und spezifischer Oberfläche unterstrichen. Zusätzlich können Metalle der III. bis VI. Gruppe des Periodensystems verwendet werden, doch werden nur Vanadium und Titan erwähnt.

Die DE-PS 1 286 001 beschreibt die Verdünnung der einzusetzenden Formamide durch Fettsäurenitrile, substituierte Benzonitrile oder den Formylverbindungen von primären aliphatischen oder aromatischen Aminen. Die weiteren Merkmale entsprechen der vorgenannten Veröffentlichungen in Katalysator und Reaktionsbedingungen. Elemente der III. bis VI. Gruppe werden erwähnt, aber nur Titan als Beispiel gezeigt.

Alle diese Verfahren befriedigen mit Bezug auf Einfachheit und Wirtschaftlichkeit des Betriebs sowie Ausbeute an Endstoff nicht.

Es wurde nun gefunden, daß man Nitrile der Formel

$$[NC]_n-R-CN \qquad (I)$$

worin R einen aliphatischen, cycloaliphatischen, araliphatischen oder aromatischen Rest bezeichnet und n 0 oder 1 bedeutet, durch Umsetzung von Formamiden bei höherer Temperatur vorteilhaft erhält, wenn man N-Formylamine der Formel

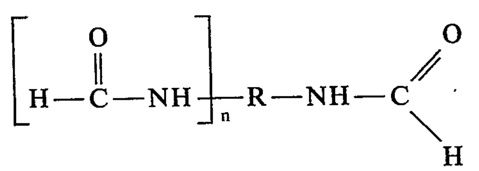

(II)

worin R und n die vorgenannte Bedeutung besitzt, bei einer Temperatur von 400 bis 700°C in Gegenwart eines oberhalb 10 bis 50 Gewichtsprozent Boroxid enthaltenden Trägerkatalysators umsetzt.

Die Umsetzung kann für den Fall der Verwendung von N,N-Diformyl-1,6-diaminohexan durch die folgenden Formeln beschrieben werden:

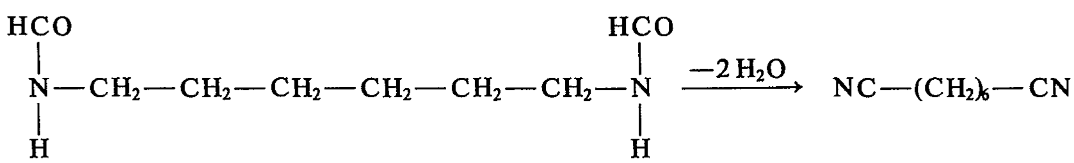


Im Vergleich mit den bekannten Verfahren liefert das Verfahren nach der Erfindung auf einfacherem und wirtschaftlicherem Wege Nitrile in besserer Ausbeute und Reinheit. Die erfindungsgemäßen Katalysatoren sind billig, leicht regenerierbar und werden nicht in deutlichem Maße während einer längeren Betriebsdauer vergiftet. Alle diese vorteilhaften Eigenschaften sind im Hinblick auf den Stand der Technik überraschend.

Bevorzugte Ausgangsstoffe II und dementsprechend bevorzugte Endstoffe I sind solche, bei denen R einen Alkylrest mit 1 bis 10 Kohlenstoffatomen, einen Alkylenrest mit 3 bis 10 Kohlenstoffatomen, einen Cycloalkylrest oder Cycloalkylenrest mit je 5 bis 7 Kohlenstoffatomen, einen Aralkyl-, Alkylaryl-, Aralkylen- oder Alkylarylenrest mit jeweils 7 bis 12 Kohlenstoffatomen, Phenylrest oder Phenylenrest bedeutet und n 0 oder 1 bezeichnet. Die vorgenannten Reste können noch durch unter den Reaktionsbedingungen inerte Gruppen, z. B. Alkylgruppen mit 1 bis 4 Kohlenstoffatomen, substituiert sein.

So kommen als Ausgangsstoffe II in Frage: N-Methyl-, N-Ethyl-, N-Propyl-, N-Isopropyl-, N-Butyl-, N-sek.-Butyl-, N-Isobutyl-, N-tert.-Butyl-, N-Cyclohexyl-, N-Cyclopentyl-, N-Benzyl-, N-Phenyl-N-formylamin; entsprechende Propylen-, Isopropylen-, Butylen-, Isobutylen-, n-Butylen-, sek.-Butylen-, tert.-Butylen-, Cyclohexylen-, Aralkylen-, Alkylarylen-, Phenylengruppen mit 2 endständigen N,N'-Bisformylaminen.

Die katalytische Umsetzung wird bei einer Temperatur von 400 bis 700°C, zweckmäßig von 450 bis 600°C, vorzugsweise von 500 bis 600°C, drucklos oder unter Druck, kontinuierlich oder diskontinuierlich durchgeführt.

Gegebenenfalls können inerte Gase oder Gasgemische wie Kohlenstoffmonoxid und/oder Wasserstoff, bevorzugt Stickstoff, als Lösungs- und Verdünnungsmittel eingesetzt werden.

Vorteilhaft ist die Verwendung des bei der Umsetzung gebildeten Gasgemisches, im wesentlichen Kohlenstoffmonoxid, Kohlenstoffdioxid und Wasserstoff, durch Abtrennung aller sonstigen Reaktionskomponenten und Rückführung (Kreisgas). Zwar kann man auch kontinuierlich in einem Festbettreaktor das erfindungsgemäße Verfahren verwenden; bevorzugt wird aber ein Wirbelschichtreaktor verwendet.

Die Verwendung des Wirbelschichtreaktors gestattet es vorteilhafterweise, die Ausgangsstoffe fest oder flüssig direkt in die Reaktionszone mit den erhöhten Temperaturen einzutragen.

Daneben ist es aber für zahlreiche, z. B. aromatische Ausgangsstoffe II, ebenso wie in den bekannten Festbettreaktoren möglich, die Ausgangsstoffe gasförmig dem Reaktor zuzuführen. Eine vorteilhafte Arbeitsweise führt das gasförmige primäre Amin mit verdampftem Formamid dem Reaktor zu, wobei sich die Ausgangsstoffe II bilden.

Weiterhin wurde gefunden, daß die Beladung eines Trägerkatalysators, z. B. eines Kieselgelkatalysators, mit oberhalb 10 bis 50, zweckmäßig 11 bis 50, vorteilhaft 15 bis 40, bevorzugt 16 bis 30, insbesondere 16 bis 25 Gewichtsprozent $B_2O_3$, entsprechend bezogen auf 50 bis unterhalb 90, zweckmäßig 50 bis 89, vorteilhaft 60 bis 85, bevorzugt 70 bis 84, insbesondere 75 bis 84 Gewichtsprozent Kieselgelkatalysator, zu besseren Nitrilausbeuten führen. Die Wasserdampfflüchtigkeit des Boroxids führt bei längerem Einsatz des Katalysators zu Verlusten an $B_2O_3$ und parallel dazu an Nitrilausbeute. Es hat sich gezeigt, daß die $B_2O_3$- und Nitrilausbeuteverluste vollständig vermieden werden, wenn man eine der $B_2O_3$-Verlustrate äquivalente Menge an Borsäure den festen oder flüssigen Ausgangsstoffen zumischt.

Als Träger kommen zweckmäßig in Frage: Kieselsäureverbindungen wie Silikate, z. B. Montmorillonit, Floridaerde, Quarz, Asbest; gefällte Kieselsäure, Kieselgel, Kieselgur; Titandioxid, Zirkondioxid, Zinndioxid, Aktivkohle; Erdalkalisulfate oder Erdalkaliphosphate, z. B. die Calcium- oder Bariumsalze; Aluminiumoxid von $\alpha$- oder $\gamma$-Aluminiumoxid; oder entsprechende Gemische vorgenannter Trägermaterialien, z. B. Aluminiumsilikat, Magnesiumaluminiumsilikathydrat, Dimagnesiumaluminiumsilikathydrat, Natriumaluminiumsilikat, Calciumaluminiumsilikat, Fullererde, Tone, Bleicherden wie Bentonit, Bauxit, Bimsstein, Andalusit, Kaolin, Allophane, Zeolithe, Mullit, Korund, $\gamma$-Tonerde, Hydrargillit, Böhmit.

Die Herstellung der Trägerkatalysatoren wird nach den üblichen Verfahren, z. B. durch Auftragen von Boroxid auf den Träger, Trocknen und Calcinieren, beispielsweise zwischen 400 und 1200°C in inerter Atmosphäre, durchgeführt. Der Träger kann auch in seiner gewünschten geometrischen Form mit einer Lösung der entsprechenden Borverbindung bzw. mit Boratlösungen getränkt und getrocknet werden.

Ebenfalls kann man das Trägermaterial mit den entsprechenden Verbindungen und Wasser verkneten, in die gewünschte Form bringen, trocknen und bei einer Temperatur von 400 bis 1200°C calcinieren.

Die Teilchengröße der Katalysatoren (Boroxid + Träger Kieselgel) beträgt vorzugsweise von 0,05 bis 7, insbesondere 2 bis 4 Millimeter. Die Form kann beliebig, z. B. in Pillen-, Zylinder- oder Strangform, kugelförmig oder körnig, gewählt werden. Bevorzugt sind bei dem Katalysator auf Träger Porenvolumina von 0,05 bis 1 Milliliter je Gramm, spezifische Oberfläche von 1 bis 300 Quadratmeter je Gramm und Schüttgewichte von 0,4 bis 1 Gramm je Milliliter.

Vorzugsweise werden die Katalysatoren auf dem Träger in Splitt- oder Kugelform in der Wirbelschicht eingesetzt, wobei zweckmäßig Katalysatorteilchen mit Korngrößen von 0,05 bis 3 mm, insbesondere von 0,1 bis 1 mm, bevorzugt 0,2 bis 0,4 mm, verwendet werden. Die Schichthöhe des Katalysatorbettes im Wirbelzustand beträgt vorteilhaft 30 bis 2000 Millimeter oder wird zweckmäßig so gewählt, daß die Verweilzeiten des Ausgangsstoffes in der Katalysatorschicht von 0,01 bis 20, vorzugsweise von 1 bis 5 Sekunden ergeben.

Die für die katalytische Umsetzung zu den entsprechenden Nitrilen benötigten Formylamine können durch Reaktion der entsprechenden Amine mit Ameisensäureestern, Formamid oder Ameisensäure im

Molverhältnis 1 : 2 bis 1 : 4, vorteilhaft 1 : 2 bis 2,5, vorzugsweise bei erhöhter Temperatur und Druck, hergestellt werden.

Die Reaktion kann wie folgt durchgeführt werden: Der feste oder zweckmäßigerweise verflüssigte oder gegebenenfalls verdampfte Ausgangsstoff, vorzugsweise im Gemisch mit inerten gasförmigen Lösungs- und Verdünnungsmitteln, wird bei der Reaktionstemperatur über den Katalysator geleitet. Das aus dem Reaktor dampfförmig austretende Reaktionsgemisch wird gegebenenfalls dann in einem Zyklon entstaubt, in einer gekühlten Vorlage kondensiert. Durch fraktionierte Destillation wird zweckmäßig der Endstoff abgetrennt. Der Endstoff kann auch durch Umkristallisation oder Umfällung aus geeigneten Lösungsmitteln, z. B. mit Toluol, Dimethylformamid oder verdünnten Säuren, z. B. mit Ameisensäure, isoliert werden.

In einer bevorzugten Ausführungsform des Verfahrens wird der Ausgangsstoff in einer Wirbelschicht bei der Reaktionstemperatur umgesetzt. Der Katalysator bzw. Katalysator auf Träger kann durch Inertgas oder einem Gemisch des gasförmigen Ausgangsstoffes mit Inertgas bei Normaldruck oder vermindertem oder erhöhtem Druck in einer Wirbelschicht gehalten werden.

Entsprechend kann die Gesamtmenge oder eine Teilmenge des Ausgangsstoffes getrennt von dem Wirbelschichtgas in den Wirbelschichtreaktor eingeleitet werden.

Der Ausgangsstoff wird zweckmäßig in einem beheizten Vorratsgefäß flüssig gehalten und in einen Verdampfer dosiert, der dem Wirbelschichtreaktor vorgeschaltet ist. Gleichzeitig leitet man vorteilhaft einen schwachen Inertgasstrom durch den Verdampfer. Der verdampfte Ausgangsstoff wird zusammen mit dem Inertgasstrom durch das Katalysatorbett geleitet. Der Ausgangsstoff kann aber auch fest, vorteilhaft in einer Korngröße zwischen 0,05 bis 3 mm oder zweckmäßig aus einem beheizten Vorratsgefäß entweder allein oder zusammen mit Inertgas flüssig in den Reaktor dosiert werden.

Man kann das Verfahren nach der Erfindung in einem einfachen oder unterteilten, offenen oder geschlossenen Wirbelschichtsystem mit und ohne Fließstaubzirkulation durchführen. Bezüglich Reaktoren, Durchführung, Verfahrensvarianten und Reaktionsbedingungen des Wirbelschichtverfahrens wird auf Ullmanns Encyklopädie der technischen Chemie, Band 1, Seiten 916 ff., verwiesen.

Das aus dem Reaktor dampfförmig austretende Reaktionsgemisch wird dann in einem Zyklon entstaubt und in einer gekühlten Vorlage kondensiert. Die weitere Aufarbeitung des Reaktionsproduktes erfolgt in der vorgenannten Weise. Die Kondensation des Reaktionsgemisches kann zweckmäßigerweise auch so gestaltet werden, daß man den dampfförmigen Reaktionsaustrag in den unteren Teil einer Trennkolonne führt und in dieser Trennkolonne eine weitgehende Trennung des gewünschten Endstoffs von den gebildeten Nebenprodukten vornimmt, aus dem Sumpf der Trennkolonne den vorgereinigten Endstoff kontinuierlich oder diskontinuierlich abzieht und in vorgenannter Weise weiter reinigt, gleichzeitig über den Kopf der Kolonne die weiteren Reaktionsprodukte und das gebildete Reaktionswasser abzieht, das verbleibende inerte Reaktions- und Verdünnungsgas in eine Waschkolonne führt, anschließend trocknet und als Wirbelgas- und Verdünnungsgas (Kreisgas) zurückführt, wobei überschüssiges Gas aus dem System entfernt wird.

Die nach dem Verfahren herstellbaren Nitrile sind wertvolle Zwischenprodukte, z. B. für die Herstellung von Kunststoffen, Farbstoffen, Pflanzenschutzmitteln, Textilhilfsmitteln.

Die in den folgenden Beispielen aufgeführten Teile bedeuten Gewichtsteile. Die Gewichtsteile verhalten sich zu den Volumenteilen wie Kilogramm zu Liter.

### Beispiel 1

a) 116 Teile 1,6-Diaminohexan werden unter Rühren und Kühlung mit 320 Teilen Ameisensäuremethylester bei 25 bis 30° C gemischt. Nach dem Abkühlen des Reaktionsgemisches werden die ausgefallenen Kristalle (Fp. 110° C) abgesaugt und getrocknet.

b) 172 Teile des gebildeten N,N-Diformyldiaminohexans werden pro Stunde aus einem Vorratsgefäß zusammen mit 400 000 Volumenteilen pro Stunde Stickstoff durch einen auf 520° C erhitzten Wirbelreaktor geleitet. Der Wirbelreaktor ist ein vertikales, elektrisch beheiztes Quarzrohr, das nach unten mit einer Quarzfritte abgeschlossen ist. Der Ausgangsstoff wird als Schmelze seitlich in den Reaktor dosiert.

Das Quarzrohr ist mit 500 Teilen eines Katalysators aus 20 Teilen $B_2O_3$ auf 80 Teilen Kieselgel (Korngröße 0,1 bis 0,3 mm) zur Hälfte gefüllt.

Die Verweilzeit in der Katalysatorzone im Wirbelzustand beträgt 2 Sekunden. Die den Reaktor verlassenden Dämpfe werden kondensiert und fraktioniert destilliert. Man erhält stündlich 102 Teile Korksäuredinitril (Kp. 148° C/3 mbar) (75% der Theorie, bezogen auf das eingesetzte Diformamid).

### Beispiel 2

a) 121 Teile 2,6-Dimethylanilin und 121 Teile Toluol werden mit 69 Teilen einer 80gewichtsprozentigen wäßrigen Ameisensäurelösung bei 110° C unter Rühren umgesetzt. Nach dem Auskreisen des Reaktionswassers werden 149 Teile N-Formyl-2,6-dimethylanilin mit dem Schmelzpunkt von 169° C aus

dem Reaktionsgemisch durch Destillation isoliert.

b) 149 Teile N-Formyl-2,6-dimethylanilin werden pro Stunde mit 800 000 Volumenteilen pro Stunde Kreisgas (im wesentlichen CO) durch einen auf 570°C erhitzten Wirbelreaktor geleitet. Der Ausgangsstoff wird in Kristallform seitlich in den Reaktor eingetragen. Die Verweilzeit und der Katalysator entsprechen dem Beispiel 1b).

Die den Reaktor verlassenden Reaktionsgase werden mit Toluol gewaschen und die nicht kondensierbaren Bestandteile dem Kreisgaskompressor zugeleitet.

Aus der toluolischen Lösung werden pro Stunde durch Kristallisation 125 Teile 2,6-Dimethylbenzoesäurenitril (95% der Theorie) vom Fp. 93°C isoliert.

## Patentanspruch

Verfahren zur Herstellung von Nitrilen der Formel

$$[NC]_{n}-R-CN \qquad (I)$$

worin R einen aliphatischen, cycloaliphatischen, araliphatischen oder aromatischen Rest bezeichnet und n 0 oder 1 bedeutet, durch Umsetzung von Formamiden bei höherer Temperatur, dadurch gekennzeichnet, daß man N-Formylamine der Formel

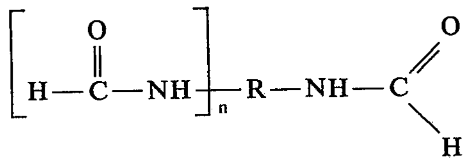

(II)

worin R und n die vorgenannte Bedeutung besitzen, bei einer Temperatur von 400 bis 700°C in Gegenwart eines oberhalb 10 bis 50 Gewichtsprozent Boroxid enthaltenden Trägerkatalysators umsetzt.

## Claim

A process for the preparation of nitriles of the formula

$$[NC]_{n}-R-CN \qquad (I)$$

where R is an aliphatic, cycloaliphatic, araliphatic or aromatic radical, and n is 0 or 1, by reacting formamides at elevated temperature, wherein N-formylamines of the formula

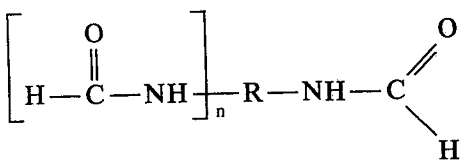

(II)

where R and n have the above meanings, are reacted at from 400 to 700°C in the presence of a supported catalyst containing from 10 to 50% by weight of boric oxide.

## Revendication

Procédé de préparation de nitriles de la formule

$$[NC]_{n}-R-CN \qquad (I)$$

dans laquelle R désigne un groupe aliphatique, cycloaliphatique, araliphatique ou aromatique et n vaut 0 ou 1, par réaction de formamides à des températures accrues, caractérisé en ce que l'on fait réagir des N-formylamines de la formule

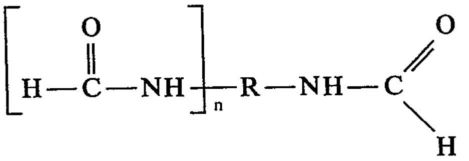

(II)

dans laquelle R et n possèdent la signification définie, à une température comprise entre 400 et 700° C en présence d'un catalyseur sur support, qui contient une proportion supérieure à 10 et pouvant aller jusqu'à 50% en poids d'oxyde de bore.